# EUROPEAN PATENT APPLICATION

(11) **EP 4 454 485 A1**
(43) Date of publication of application: **30.10.2024**
(21) Application number: 22911933.4
(22) Date of filing: 21.12.2022
(51) Int. Cl.: A23L 33/105, A61K 36/752, A61P 25/20

(54) **COMPOSITION COMPRISING EXTRACT OF ROASTED CITRUS BY-PRODUCTS AS ACTIVE INGREDIENT FOR PREVENTION, AMELIORATION, OR TREATMENT OF SLEEP DISORDER OR INSOMNIA**

(30) Priority: 21.12.2021 KR 20210184063; 15.12.2022 KR 20220175871
(71) Applicant: Nutra-IT Inc., Busan 48513 (KR)
(72) Inventor: CHO, Suengmok, Busan 48513 (KR); KIM, Seonghui, Tongyeong-si Gyeongsangnam-do 53043 (KR); KIM, Duhyeon, Busan 48499 (KR); KIM, Dongmin, Busan 48500 (KR); CHOI, Gibeom, Busan 46323 (KR); KIM, Jongmin, Gyeongsan-si Gyeongsangbuk-do 38649 (KR); CHOI, Yunjin, Busan 49418 (KR); LEE, Chahyeon, Busan 48510 (KR); LEE, Jaekwang, Jeonju-si Jeollabuk-do 54860 (KR)
(74) Representative: V.O.
(86) International application number: PCT/KR2022/020972
(87) International publication number: WO 2023/121300

(57) **Abstract**

The present disclosure relates to a composition for preventing, ameliorating or treating sleep disorder or insomnia, which contains an extract of a roasted citrus byproduct as an active ingredient.

The extract of a roasted citrus byproduct of the present disclosure has very excellent effect of decreasing sleep latency, increasing sleep duration and increasing non-REM sleep. In addition, the composition of the present disclosure, which contains ingredients derived from the naturally occurring citrus byproduct, can ensure safety without inducing the side effects of synthetic drugs including pentobarbital and benzodiazepine and with no side effects such as cognitive impairment, tolerance or dependency even after long-term use. Accordingly, the composition for preventing, alleviating or treating sleep disorder, which contains an extract of a roasted citrus byproduct as an active ingredient, of the present disclosure is very useful for preparation of a food composition and a pharmaceutical composition.

## Description

### [Technical Field]

The present disclosure relates to a composition for preventing, ameliorating or treating sleep disorder or insomnia, which contains an extract of a roasted citrus byproduct as an active ingredient.

### [Background Art]

Although sleep can be defined in many aspects, it is mainly defined as a state in which the ability of recognizing the external environment and responding thereto is reduced reversibly, repeatedly and normally with reduced physical activity. Although sleep is related with brain activities, it is also deeply related with the physiological changes of other body parts.

Sleep disorder is one of the problems that afflict moderns. It is a very common disease that about 20% or more of the population have suffered or are suffering. The sleep disorder refers to a state of lack of sound sleep, inability to keep awakened during daytime, or trouble during sleeping or waking because of an irregular sleep rhythm. In particular, insomnia refers to a state in which the state of unsatisfactory sleep quantity and quality lasts for a very long time. The symptoms of insomnia include difficulty falling asleep, waking up easily, being unable to return to sleep, waking up too early in the morning, napping, waking during the night, failing to have a sound sleep, unsleepiness, frequent dreaming, etc.

Sleep disorder can cause various individual and social problems and also causes learning disorder, lowering of work efficiency, traffic accidents, safety accidents, emotional disturbance, social isolation, unsatisfactory marriage life, industrial disasters, etc. In addition, unless treated properly, sleep disorder may worsen or delay the recovery of existing internal, neurological or mental diseases and may cause severe diseases such as myocardial infarction, stroke. According to recent data, 43% of people suffer from sleep disorder and the incidence rate of insomnia is as high as 15%.

For treatment of sleep disorder, chemical sleeping drugs are used to induce and maintain sleep by reversibly suppressing the central nervous system. The existing sleeping drugs suppress the central nervous system like anesthetics. They have sedative effects at low doses and induce sleep at moderate doses, but can cause coma and paralysis or decrease respiration at high doses. Barbital-based drugs may have tolerance and dependency problems due to low safety and can cause sleep disorder due to nightmares, etc. if use is stopped after long-term medication.

Recently, benzodiazepine-based drugs with anti-anxiety activity and less harmful effects are used. Many drugs including benzodiazepine exhibit pharmacological activity by binding to the GABA_{A} receptor. When a drug or an adjuvant binds to the benzodiazepine site, the affinity of the GABA_{A} receptor for GABA is enhanced, and the effect of relieving anxiety, alleviating spasm and inducing sedation and sleep is exhibited as the influx of chloride ions into cells is increased. The GABA_{A} receptor is a pentameric protein that constitutes a membrane ion channel. It is closely related to the regulation of sedation, sleep, anxiety, muscle tone, spasm, memory loss, etc. through the action of GABA (γ-aminobutyric acid). Long-term use of these drugs leads to side effects such as tolerance and dependency as well as symptoms such as muscular atrophy, amnesia, lethargy, coma, coronary vasodilation, myocardial blockage, etc. In particular, use in pregnancy can cause birth defects.

Accordingly, the necessity and demand of natural sleep aids that can replace sleeping drugs for long-term users and sleep-enhancing functional health foods are increasing.

### [Disclosure]

### [Technical Problem]

The present disclosure is directed to providing a composition consisting of naturally derived ingredients, which is capable of effectively preventing, alleviating or treating sleep disorder or insomnia while ensuring safety without inducing the side effects of synthetic drugs including pentobarbital and benzodiazepine.

Specifically, the present disclosure is directed to providing a food composition capable of preventing or alleviating sleep disorder or insomnia, which contains an extract of a roasted citrus byproduct as an active ingredient.

The present disclosure is also directed to providing a pharmaceutical composition capable of preventing or treating sleep disorder or insomnia, which contains an extract of a roasted citrus byproduct as an active ingredient.

The present disclosure is also directed to providing a functional health food composition capable of preventing or alleviating sleep disorder or insomnia, which contains an extract of a roasted citrus byproduct as an active ingredient.

The present disclosure is also directed to providing a method for preparing a citrus byproduct extract with enhanced effect of preventing or alleviating sleep disorder or insomnia.

### [Technical Solution]

A food composition for preventing or alleviating sleep disorder or insomnia of the present disclosure may contain an extract obtained by extracting a roasted citrus byproduct with a C₁₋₆ lower alcohol or an aqueous solution thereof as an active ingredient.

The citrus may be one or more selected from lemon, lime, tangerine, hallabong, cheonhyehyang, green tangerine, kumquat, orange, grapefruit, hardy orange and citron.

The roasting may be performed at 150-230 °C for 5-20 minutes.

The citrus byproduct may be one or more selected from the rind and pomace of a citrus.

The extract of the citrus byproduct may be obtained by extracting with 10-90 vol% methanol, ethanol, butanol, propanol or an aqueous solution thereof.

The extract of the citrus byproduct may be obtained by extracting with a 50-80 vol% ethanol aqueous solution.

The extract of the citrus byproduct may be obtained by extracting at 10-80 °C.

The extract of the citrus byproduct may be prepared by a method including: (1) a step of drying and pulverizing a byproduct of a citrus; (2) a step of roasting the pulverization product of the citrus byproduct; (3) a step of extracting the roasted pulverization product of the citrus byproduct with a C₁₋₆ lower alcohol or an aqueous solution thereof; and (4) a step of filtering and concentrating the extract of the citrus byproduct.

The prevention or alleviation of sleep disorder or insomnia may be for decreasing sleep latency, increasing sleep duration or increasing non-REM sleep.

The food composition may be in the form of a powder, a granule, a tablet, a capsule or a beverage.

A pharmaceutical composition for preventing or treating sleep disorder or insomnia of the present disclosure may contain an extract obtained by extracting a roasted citrus byproduct with a C₁₋₆ lower alcohol or an aqueous solution thereof as an active ingredient.

In addition, a functional health food composition for preventing or alleviating sleep disorder of the present disclosure may contain an extract obtained by extracting a roasted citrus byproduct with a C₁₋₆ lower alcohol or an aqueous solution thereof as an active ingredient.

In addition, a method for preparing a citrus byproduct extract with enhanced effect of preventing or alleviating sleep disorder or insomnia of the present disclosure may include: (1) a step of drying and pulverizing a byproduct of a citrus; (2) a step of roasting the pulverization product of the citrus byproduct; (3) a step of extracting the roasted pulverization product of the citrus byproduct with a C₁₋₆ lower alcohol or an aqueous solution thereof; and (4) a step of filtering and concentrating the extract of the citrus byproduct.

### [Advantageous Effects]

An extract of a roasted citrus byproduct of the present disclosure has excellent effect of decreasing sleep latency, increasing sleep duration and increasing non-REM sleep. In addition, the composition of the present disclosure, which contains ingredients derived from the naturally occurring citrus byproduct, can ensure safety without inducing the side effects of synthetic drugs including pentobarbital and benzodiazepine and with no side effects such as cognitive impairment, tolerance or dependency even after long-term use. Accordingly, the composition for preventing, alleviating or treating sleep disorder, which contains an extract of a roasted citrus byproduct as an active ingredient, of the present disclosure is very useful for preparation of a food composition and a pharmaceutical composition.

### [Brief Description of Drawings]

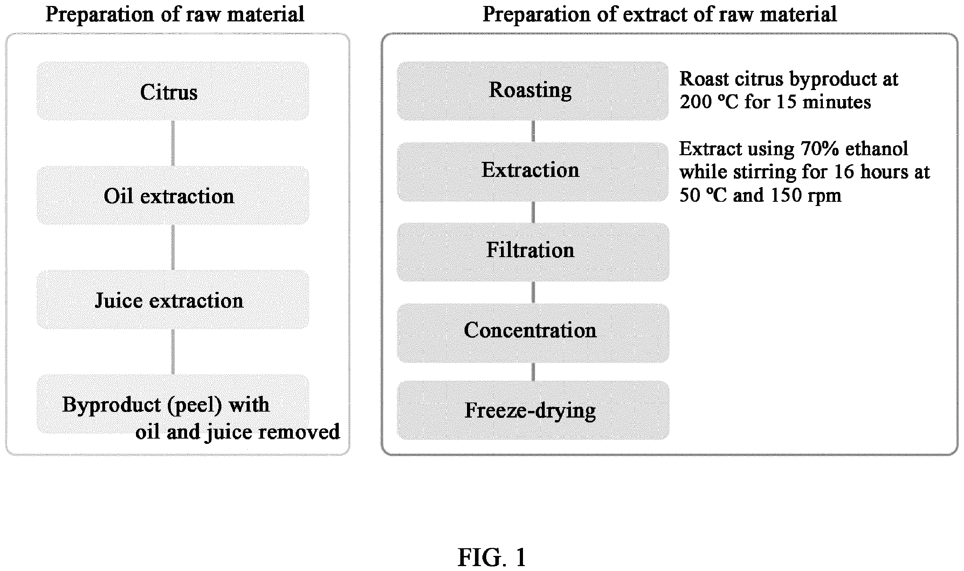

FIG. 1 is a schematic diagram which briefly illustrates a process of preparing an extract of a roasted citrus byproduct according to an exemplary embodiment of the present disclosure.

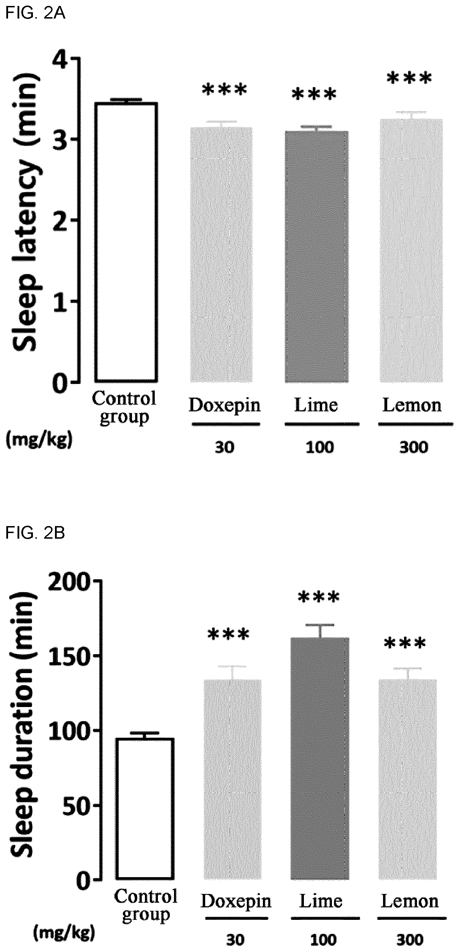

FIG. 2a compares the sleep latency-decreasing effect of an extract of Example 1 (lime) and an extract of Example 2 (lemon) in mice to which a sleeping dose of pentobarbital (45 mg/kg, i.p.) was administered with doxepin hydrochloride (DH), which is a representative sleeping drug, and FIG. 2b compares the sleep duration-increasing effect of an extract of Example 1 (lime) and an extract of Example 2 (lemon) in mice to which a sleeping dose of pentobarbital (45 mg/kg, i.p.) was administered with doxepin hydrochloride (DH). For a control group (5% Tween 80-saline 10 mL/kg), doxepin hydrochloride (DH, 30 mg/kg), an extract of Example 1 (lime, 100 mg/kg) or an extract of Example 2 (lemon, 300 mg/kg) was administered orally (p.o.) and pentobarbital was administered 45 minutes later. Each graph represents mean values (mean ± SEM, n = 10). *** indicates significant difference from the control group with p < 0.001 (Tukey's test).

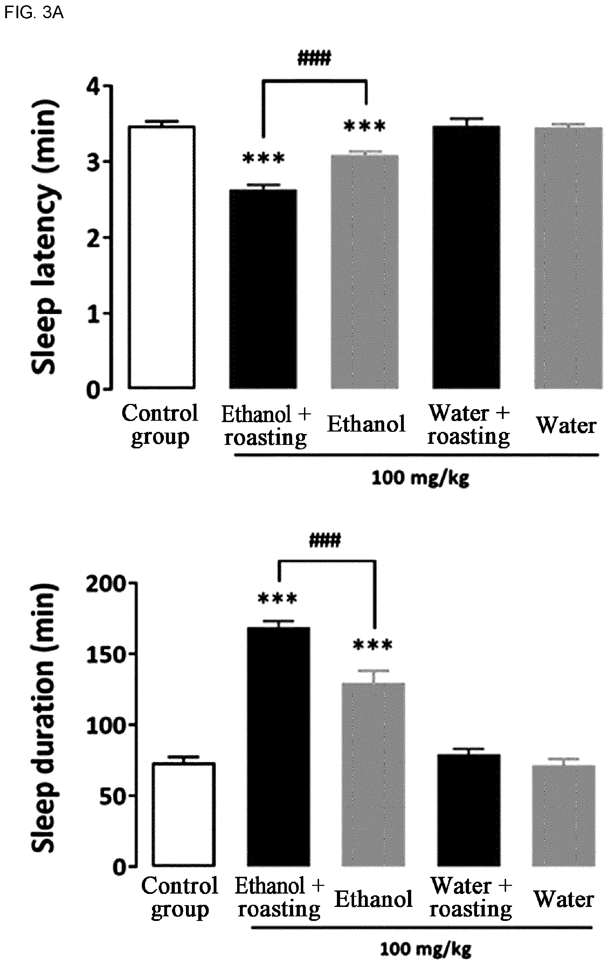

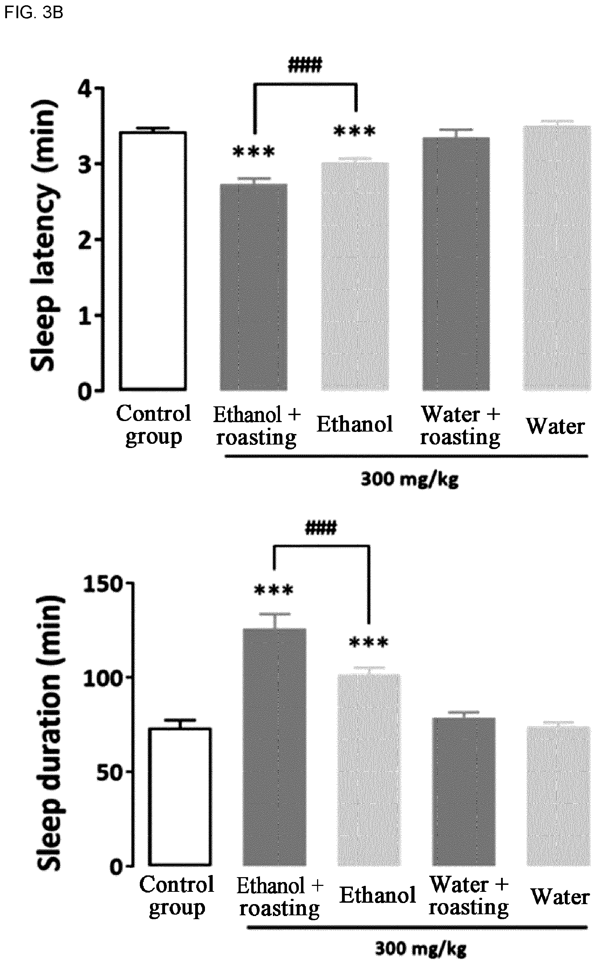

FIG. 3a compares the effect of an extract of Example 1 (lime) on sleep latency and sleep duration in mice to which a sleeping dose of pentobarbital (45 mg/kg, i.p.) was administered with Comparative Example 1-1 wherein roasting was omitted, Comparative Example 2-1 wherein hot water extraction was conducted instead of ethanol extraction after roasting and Comparative Example 3-1 wherein hot water extraction was conducted only, and FIG. 3b compares the effect of an extract of Example 2 (lemon) on sleep latency and sleep duration in mice to which a sleeping dose of pentobarbital (45 mg/kg, i.p.) was administered with Comparative Example 1-2 wherein roasting was omitted, Comparative Example 2-2 wherein hot water extraction was conducted instead of ethanol extraction after roasting and Comparative Example 3-2 wherein hot water extraction was conducted only. For a control group (5% Tween 80-saline 10 mL/kg), an extract of Example 1 (lime, EtOH + roasting), an extract of Example 2 (lemon, EtOH + roasting), extracts of Comparative Example 1-1 and Comparative Example 1-2 (EtOH), extracts of Comparative Example 2-1 and Comparative Example 2-2 (water + roasting) or extracts of Comparative Example 3-1 and Comparative Example 3-2 (water) was administered orally (p.o.) and pentobarbital was administered 45 minutes later. Each graph represents mean values (mean ± SEM, n = 10). *** indicates significant difference from the control group with p < 0.001, and ### indicates significant difference from the extract of Comparative Example 1-1 or Comparative Example 1-2 (EtOH) with p < 0.001 (Tukey's test).

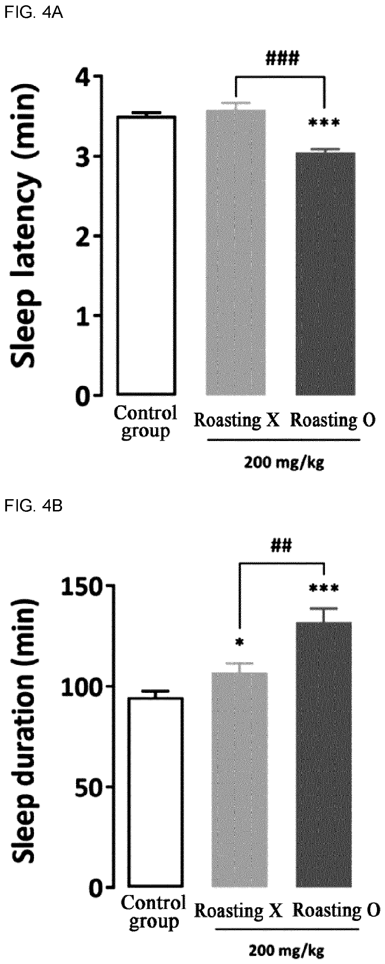

FIG. 4a compares the effect of an extract of Example 3 (kumquat) on sleep latency in mice to which a sleeping dose of pentobarbital (45 mg/kg, i.p.) was administered with Comparative Example 1-3 wherein roasting was omitted, and FIG. 4b compares the effect of an extract of Example 3 (kumquat) on sleep duration in mice to which a sleeping dose of pentobarbital (45 mg/kg, i.p.) was administered with Comparative Example 1-3 wherein roasting was omitted. For a control group (5% Tween 80-saline 10 mL/kg), an extract of Example 3 (roasting O) or an extract of Comparative Example 1-3 (roasting X) was administered orally (p.o.) and pentobarbital was administered 45 minutes later. Each graph represents mean values (mean ± SEM, n = 10). *** indicates significant difference from the control group with p < 0.001, and ### indicates significant difference from Comparative Example 1-3 with p < 0.001 (Tukey's test).

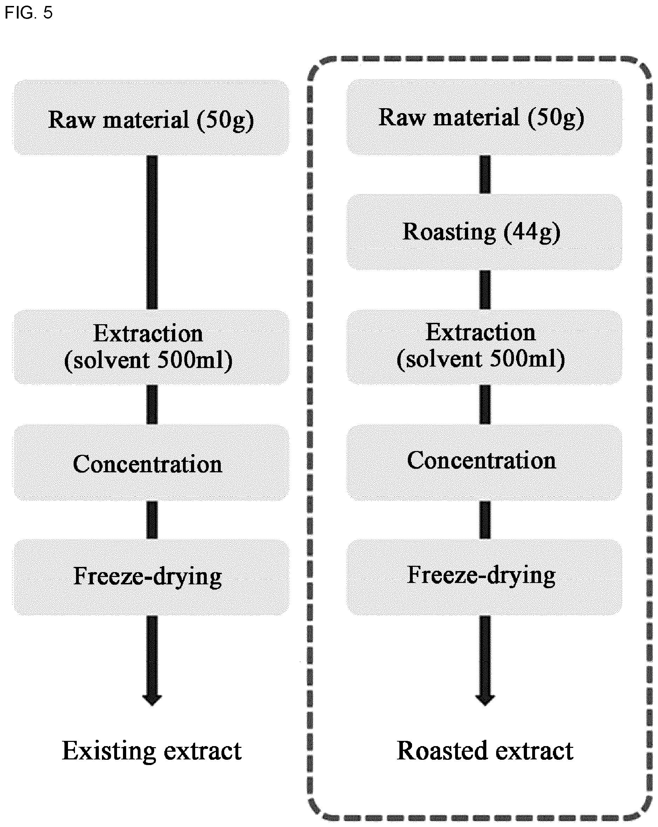

FIG. 5 briefly compares the amount of a raw material used in a method of preparing an extract according to an example of the present disclosure with the amount of a raw material used in a method of a comparative example without roasting.

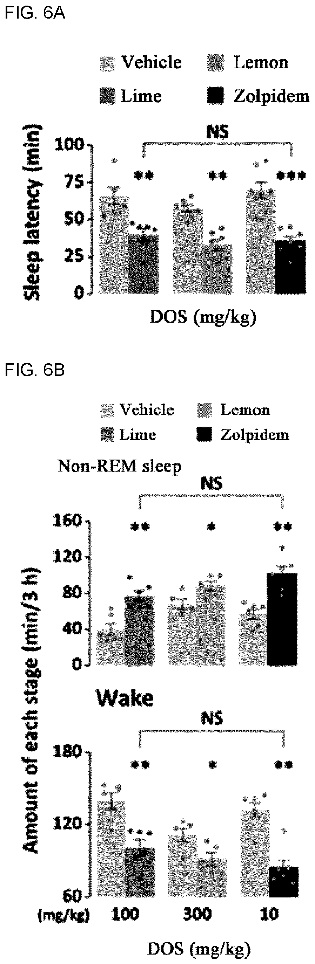

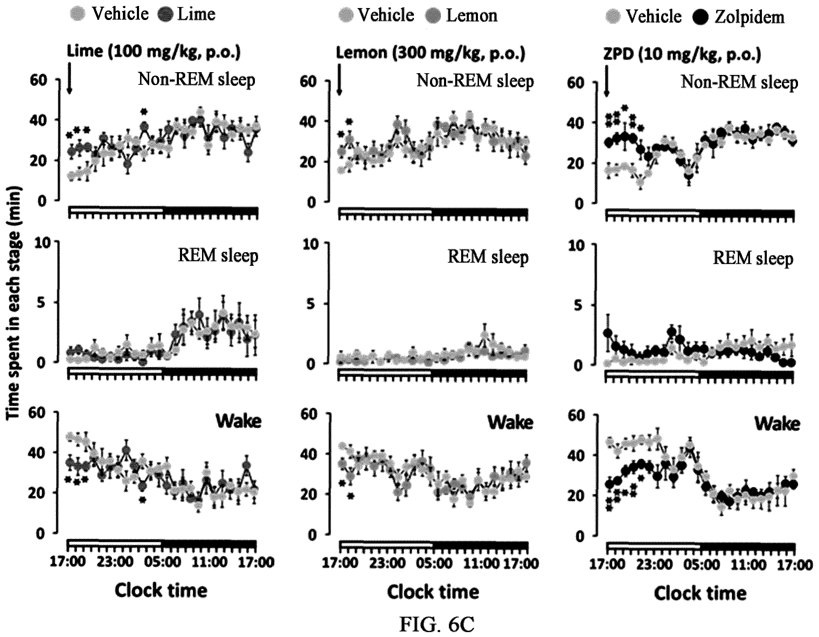

FIG. 6a compares the effect of a control group (vehicle), an extract of Example 1 (lime) and an extract of Example 2 (lemon) on decreased sleep latency in mice through sleep architecture analysis with zolpidem (ZPD), which is a commercially available sleeping drug, FIG. 6b compares the effect of a control group (vehicle), an extract of Example 1 (lime) and an extract of Example 2 (lemon) on increased non-REM sleep (NREMS) and decreased wake with zolpidem (ZPD), which is a commercially available sleeping drug, and FIG. 6c compares the effect of a control group (vehicle), an extract of Example 1 (lime) and an extract of Example 2 (lemon) on the change in non-REM sleep (NREMS), REM sleep (REMS) and wake for 24 hours with zolpidem (ZPD), which is a commercially available sleeping drug. Vehicle indicates the control group, * indicates significant difference from the control group with p < 0.05, ** indicates significant difference from the control group with p < 0.01, and *** indicates significant difference from the control group with p < 0.001 (t-test). Wake indicates wakefulness, NREMS indicates non-rapid eye movement sleep, and REMS indicates rapid eye movement sleep.

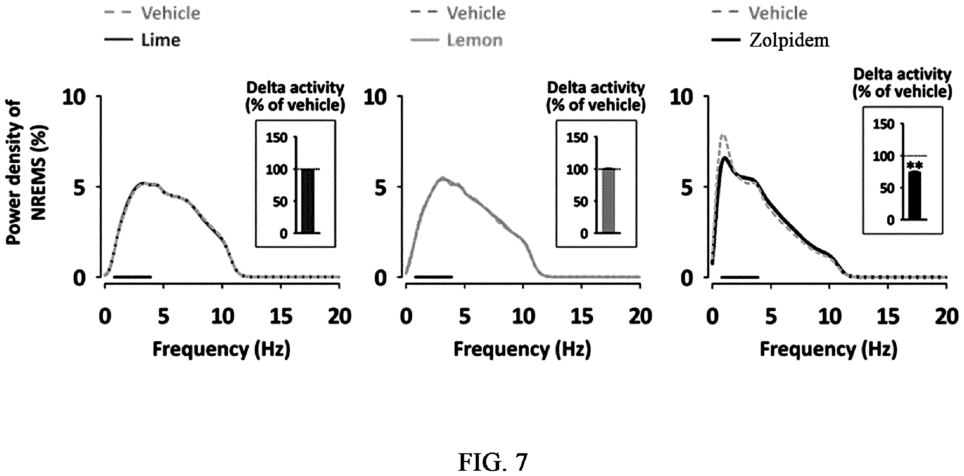

FIG. 7 shows the power density and delta activity in electroencephalograms (EEGs) during non-REM sleep for an extract of Example 1 (lime, 100 mg/kg), an extract of Example 2 (lemon, 300 mg/kg) and zolpidem (ZPD, 10 mg/kg). Vehicle indicates a control group, * indicates significant difference from the control group with p < 0.05, and ** indicates significant difference from the control group with p < 0.01 (t-test). NREMS indicates non-rapid eye movement sleep.

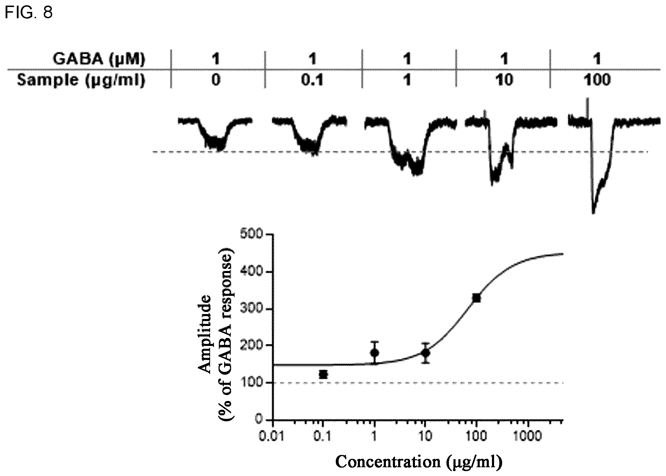

FIG. 8 shows a result of treating transformed HEK-293T cells expressing the GABA receptor with an extract of Example 1 (N1) and then measuring the activity of the GABA receptor depending on the concentration of the extract of Example 1 (N1).

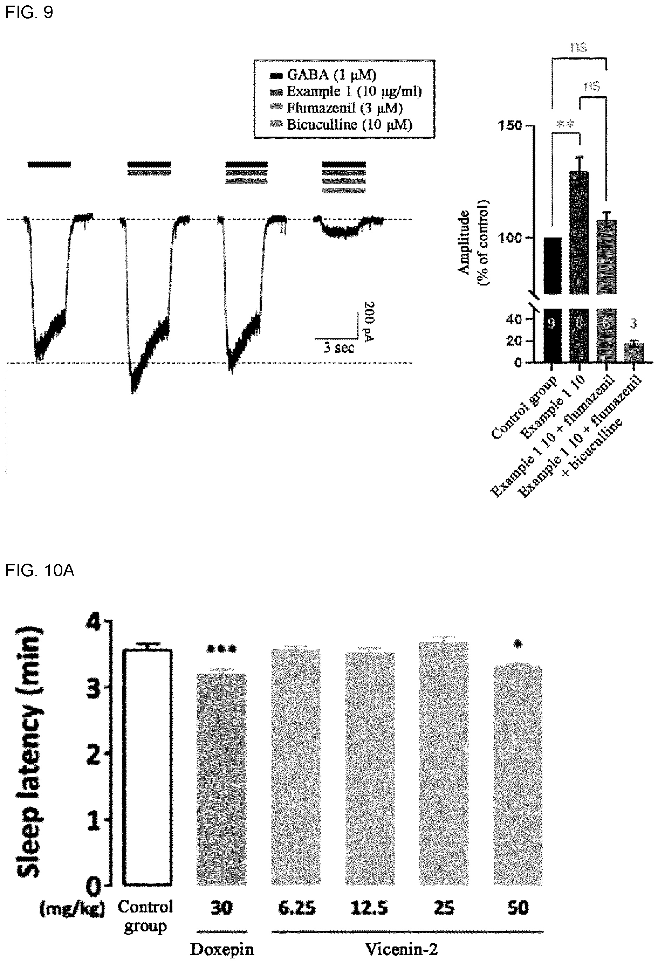

FIG. 9 shows the activity of the GABA receptor depending on treatment with an extract of Example 1 (N1 10) at a concentration of 10 µg/mL, the extract of Example 1 at a concentration of 10 µg/mL and 3 µM flumazenil (N1 10 + FLZ), and the extract of Example 1 at a concentration of 10 µg/mL, 3 µM flumazenil and 10 µM bicuculline (N1 10 + FLZ + BIC). ** indicates significant difference from a control group with p < 0.01 (t-test).

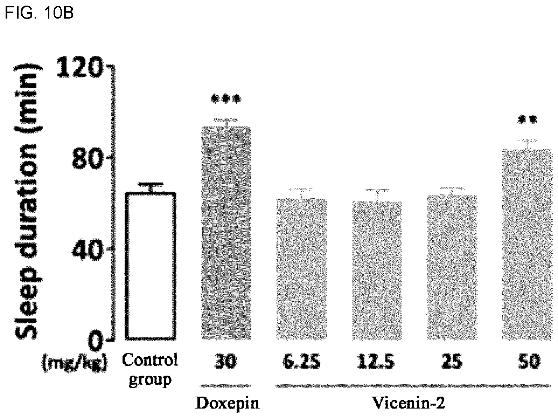

FIG. 10a compares the sleep latency-decreasing effect of vicenin in mice to which a sleeping dose of pentobarbital (45 mg/kg, i.p.) was administered with doxepin hydrochloride (DH), which is a representative sleeping drug, and FIG. 10b compares the sleep duration-increasing effect of vicenin in mice to which a sleeping dose of pentobarbital (45 mg/kg, i.p.) was administered with doxepin hydrochloride (DH). * indicates significant difference from a control group with p < 0.05, ** indicates significant difference from a control group with p < 0.01, and *** indicates significant difference from a control group with p < 0.001 (t-test).

### [Best Mode]

The present disclosure relates to a composition capable of preventing, ameliorating or treating sleep disorder or insomnia, which contains an extract of a roasted citrus byproduct as an active ingredient.

The prevention, amelioration or treatment of sleep disorder or insomnia may refer to the effect of decreasing sleep latency, increasing sleep duration or increasing non-REM sleep, although not being limited thereto.

The sleep disorder refers to a disrupted sleep pattern caused by various reasons including dysfunctional sleep mechanisms, abnormalities in physiological functions during sleep and disturbances induced by factors extrinsic to the sleep process. The sleep disorder may be, for example, insomnia. The insomnia may include middle-of-the-night insomnia, late night insomnia, prolonged awakening after sleep onset, sleep maintenance insomnia, and insomnia that follows middle-of-the-night awakening.

In the examples that will be described below, it was confirmed that the extract of a roasted citrus byproduct exhibits an effect of decreasing sleep latency and increasing sleep duration, which is comparable to or improved over that of doxepin hydrochloride or diazepam that have been used as sleeping drugs, and increases non-REM sleep time. Accordingly, the extract of a roasted citrus byproduct of the present disclosure may be used as an active ingredient of a composition for preventing, ameliorating or treating sleep disorder. Unlike the existing sleeping drugs, the extract of a roasted citrus byproduct is derived from a natural product and exhibits very superior effect of inducing and maintaining sleep with no side effect. Therefore, it can be used usefully for preventing, ameliorating or treating sleep disorder.

Hereinafter, the present disclosure is described in detail.

The composition for preventing, alleviating or treating sleep disorder of the present disclosure contains an extract of a roasted citrus byproduct as an active ingredient.

In general, the "citrus" refers to plants belonging to the genera *Citrus, Fortunella* and *Poncirus* in the subfamily *Aurantioideae* of the family *Rutaceae.* Examples of the fruits of the citrus include lemon, lime, tangerine, hallabong, cheonhyehyang, green tangerine, kumquat, orange, grapefruit, hardy orange, citron, etc. Generally, only the pulp or juice of the citrus is used to avoid astringent and sour taste. For this reason, over 50,000 tons of byproducts such as the rind, pomace, etc. of the citrus are produced in the process of preparing processed products such as beverages, chocolates, etc. Although it is known that the byproducts of the citrus are rich in flavonoids, pectins, oils, etc., most of them are being discarded.

The citrus of the present disclosure may be one or more selected from lemon, lime, tangerine, hallabong, cheonhyehyang, green tangerine, kumquat, orange, grapefruit, hardy orange and citron, specifically one or more selected from lime, lemon, tangerine and kumquat, more specifically one or more selected from lime, lemon and kumquat, more specifically one or more selected from lime and lemon.

The citrus byproduct of the present disclosure may be one or more selected from the rind and pomace of the citrus, which are obtained during the preparation of processed citrus products such as beverages, chocolates, etc. Specifically, it may be the rind of the citrus. In addition, the rind of the citrus may specifically be a byproduct obtained after extracting oil ingredients from the rind of the citrus in terms of the effect of improving sleep efficiency.

The extract of a citrus byproduct of the present disclosure may be obtained by extracting the citrus byproduct which has or has not been roasted. Specifically, the extract may be obtained after roasting in terms of the effect on sleep disorder or insomnia. In an example of the present disclosure wherein a sleeping dose of pentobarbital was administered to mice, the extract of a roasted citrus byproduct showed significant sleep latency-decreasing effect and sleep duration-increasing effect as compared to the extract of an unroasted citrus byproduct (see FIG. 3a and FIG. 3b).

The roasting of the citrus byproduct may be performed at 150-230 °C, specifically at 180-220 °C, more specifically at 190-210 °C, for 5-20 minutes, specifically for 10-20 minutes, more specifically for 12-18 minutes, more specifically for 14-16 minutes, in terms of the effect of improving sleep efficiency. If the roasting temperature is below the lower limit, the effect of improving sleep efficiency by the roasted citrus byproduct may not be achieved. And, if the roasting temperature exceeds the upper limit, the citrus byproduct may burn. If the roasting time is shorter than the lower limit, the citrus byproduct may not be roasted enough. And, if the roasting time exceeds the upper limit, the effect of improving sleep efficiency by the roasted citrus byproduct may be decreased on the contrary and the cost for commercial use may be increased.

The roasted citrus byproduct may be prepared by prepared by mixing with an extraction solvent at a weight ratio of 1 : 5-20, specifically 1 : 5-15, more specifically 1 : 8-12, performing extraction at 10-80 °C, specifically at 20-70 °C, more specifically at 40-60 °C, more specifically at 45-55 °C, for 2-30 hours, specifically for 10-20 hours, more specifically for 14-18 hours, and then performing filtration and concentration under reduced pressure. If the weight ratio of the roasted citrus byproduct and the extraction solvent is outside the above range, the active ingredients of the roasted citrus byproduct may be extracted in a small amount. If the extraction temperature is below the lower limit, the active ingredients for improving sleep efficiency may not be extracted well. And, if the extraction temperature exceeds the upper limit, the extract may be denatured. If the extraction time is shorter than the lower limit, some of the active ingredients may not be extracted. And, if the extraction time exceeds the upper limit, impurities may be extracted together with the active ingredients.

The extraction solvent may be a C₁₋₆ lower alcohol or an aqueous solution thereof. In terms of the improvement of sleep efficiency, the extraction solvent may be specifically a 10-90 vol% C₁₋₆ lower alcohol aqueous solution, more specifically a 20-80 vol% C₁₋₆ lower alcohol aqueous solution, more specifically 20-80 vol% methanol, ethanol, butanol, propanol or an aqueous solution thereof, more specifically 50-80 vol% methanol, ethanol, butanol, propanol or an aqueous solution thereof.

More specifically, an extract extracted with a 50-80 vol% ethanol aqueous solution as the extraction solvent is more preferred for prevention, alleviation or treatment of sleep disorder or insomnia.

The roasted citrus byproduct extract may be a fraction obtained by fractionating the extract obtained by extracting a roasted citrus byproduct with a C₁₋₆ lower alcohol or an aqueous solution thereof with a second organic solvent. Specifically, the citrus byproduct extract may be a fraction obtained by fractionating the ethanol extract of the roasted citrus byproduct with hexane, ethyl acetate, butanol or water.

In the present specification, the term "extract" used while mentioning the roasted citrus byproduct includes not only a crude extract obtained by treating with the extraction solvent but also a processed product of the roasted citrus byproduct extract. For example, the roasted citrus byproduct extract may be prepared into a powder through additional processes such as vacuum filtration, freeze-drying, spray drying, etc.

In a specific exemplary embodiment, the extract of a citrus byproduct of the present disclosure may be prepared by a method including: (1) a step of drying and pulverizing a byproduct of a citrus; (2) a step of roasting the pulverization product of the citrus byproduct; (3) a step of extracting the roasted pulverization product of the citrus byproduct with a C₁₋₆ lower alcohol or an aqueous solution thereof; and (4) a step of filtering and concentrating the extract of the citrus byproduct. It was confirmed that the byproduct of a citrus of the step (1) has enhanced sleep efficiency when the byproduct is obtained after extracting oil ingredients from the rind of the citrus as compared to one from which the oil ingredients have not been extracted.

In the present specification, the term "containing as an active ingredient" means that the extract of the roasted citrus byproduct is contained in an amount sufficient to achieve the desired effect or activity. A daily administration dose of the extract of the roasted citrus byproduct of the present disclosure may be 10-1,000 mg/kg, specifically 25-500 mg/kg, more specifically 50-500 mg/kg, more specifically 100-500 mg/kg, more specifically 100-300 mg/kg. If the administration dose of the extract of the roasted citrus byproduct of the present disclosure is smaller than the lower limit, the desired sleep efficiency may not be achieved. And, if the administration dose exceeds the upper limit, the sleep efficiency may not be improved enough when considering the increased administration dose. Since the extract of the roasted citrus byproduct is a natural product and has no side effect on the human body even when administered in an excess amount, the upper limit of the amount of the extract contained in the composition of the present disclosure may be selected within a proper range by those skilled in the art.

Whereas the plant extracts previously known to exhibit sleep efficiency, such as bangpung extract, paddle weed extract, rice bran extract, etc., show significant sleep efficiency only at concentrations of 500 mg/kg or higher, the extract of the roasted citrus byproduct of the present disclosure can exhibit significant sleep efficiency at a relatively low concentration.

In a pentobarbital-induced sleep test of an example of the present disclosure, it was confirmed that the extract of the roasted citrus byproduct of the present disclosure has an effect of ameliorating sleep disorder or insomnia since it decreased sleep latency and increased sleep duration when administered to a mouse model (see FIG. 2 and FIG. 3). This suggests that the extract of the roasted citrus byproduct of the present disclosure may be usefully used for preventing, ameliorating or treating sleep disorder or insomnia.

In the present disclosure, the term "prevention" refers to any action of inhibiting or delaying sleep disorder or insomnia.

In the present disclosure, the term "treatment" refers to reversal or amelioration of the symptoms of sleep disorder or insomnia or inhibition or prevention thereof, unless specified otherwise.

In the present disclosure, the term "amelioration" refers to at least decreasing parameters related with the condition to be treated by administering a composition containing the extract of the roasted citrus byproduct of the present disclosure, e.g., the severity of symptoms.

A food composition according to the present disclosure may be formulated into a powder, a granule, a tablet, a capsule, etc. using a sitologically appropriate and physiologically acceptable adjuvant for use as a functional food. As the adjuvant, an excipient, a disintegrant, a sweetener, a binder, a coating agent, a swelling agent, a lubricant, a glidant, a flavorant, etc. may be used.

In addition, the food composition according to the present disclosure may in the form of, for example, a beverage, an alcohol beverage, confectionery, a diet bar, a dairy product, meat, chocolate, pizza, ramen, other noodles, gum, ice cream, etc.

The food composition of the present disclosure may contain an ingredient commonly added for preparation of food, e.g., a protein, a carbohydrate, a fat, a nutrient, a seasoning and a flavorant, in addition to the extract of the roasted citrus byproduct as an active ingredient. The carbohydrate may be, for example, a common sugar such as a monosaccharide, e.g., glucose, fructose, etc., a disaccharide, e.g., maltose, sucrose, oligosaccharides, etc., and a polysaccharide, e.g., dextrin, cyclodextrin, etc. or a sugar alcohol such as xylitol, sorbitol, erythritol, etc. As the flavorant, a natural flavorant [thaumatin, stevia extract (e.g., rebaudioside A, glycyrrhizin, etc.)] or a synthetic flavorant (saccharine, aspartame, etc.) may be used. For example, when the food composition of the present disclosure is prepared into a drink or a beverage, citric acid, fructose syrup, sugar, glucose, acetic acid, malic acid, fruit juice, plant extract, etc. may be further added in addition to the extract of the roasted citrus byproduct of the present disclosure. The food composition may be in the form of a powder, a granule, a tablet, a capsule or a beverage.

The content of the extract of the roasted citrus byproduct in the food composition may be 0.001-20 wt%, specifically 0.001-10 wt%, 0.001-1 wt%, more specifically 0.001-0.5 wt%, more specifically 0.002-0.3 wt%, more specifically 0.005-0.3 wt%, more specifically 0.01-0.2 wt%, more specifically 0.02-0.1 wt%.

The present disclosure also provides a pharmaceutical composition for preventing or treating sleep disorder or insomnia, which contains the extract of the roasted citrus byproduct as an active ingredient.

The matters described above with regard to the "extract of a roasted citrus byproduct" will be omitted to avoid unnecessary redundancy.

The pharmaceutical composition of the present disclosure may be prepared using a pharmaceutically appropriate and physiologically acceptable adjuvant in addition to the active ingredient. As the adjuvant, an excipient, a disintegrant, a sweetener, a binder, a coating agent, a swelling agent, a lubricant, a glidant, a flavorant, etc. may be used.

The pharmaceutical composition may be formulated using one or more pharmaceutically acceptable carrier in addition to the active ingredient for adm inistration.

The pharmaceutical composition may be formulated a granule, a powder, a tablet, a coated tablet, a capsule, a suppository, a liquid, a syrup, a juice, a suspension, an emulsion, a medicinal drop, an injectable solution, etc. For example, for formulation into a tablet or a capsule, the active ingredient may be bound to a nontoxic, pharmaceutically acceptable inert carrier for oral administration such as ethanol, glycerol, water, etc. In addition, if desired or necessary, a suitable binder, lubricant, disintegrant and colorant may be added as a mixture. A suitable binder includes but is not limited to a natural sugar such as starch, gelatin, glucose or β-lactose, a corn sweetener, a natural or synthetic gum such as acacia, tragacanth or sodium oleate, sodium stearate, magnesium stearate, sodium benzoate, sodium acetate, sodium chloride, etc. The disintegrant includes but is not limited to starch, methyl cellulose, agar, bentonite, xanthan gum, etc.

When the composition is formulated into a liquid solution, one or more of saline, sterile water, Ringer's solution, buffered saline, albumin injection solution, dextrose solution, maltodextrin solution, glycerol and ethanol may be used as a sterilized, biocompatible, pharmaceutically acceptable carrier. If necessary, other common additives such as an antioxidant, a buffer, a bacteriostat, etc. may be added. In addition, an injectable formulation such as an aqueous solution, a suspension, an emulsion, a pill, a capsule, a granule or a tablet may be prepared by further adding a diluent, a dispersant, a surfactant, a binder and a lubricant.

The composition may be formulated properly depending on the corresponding disease or ingredients using the method described in Remington's Pharmaceutical Science, Mack Publishing Company, Easton PA.

The pharmaceutical composition of the present disclosure may be administered orally or parenterally. The parenteral administration may be made by intravenous injection, subcutaneous injection, intramuscular injection, intraperitoneal injection, transdermal administration, etc. Specifically, it may be administered orally.

An adequate administration dose of the pharmaceutical composition of the present disclosure varies depending on factors such as formulation method, administration type, the age, body weight, sex, pathological condition and diet of a patient, administration time, administration route, excretion rate and response sensitivity. An ordinarily skilled physician can easily determine and prescribe an administration dose effective for the desired treatment or prevention. According to a specific exemplary embodiment of the present disclosure, a daily administration dose of the pharmaceutical composition of the present disclosure may be 0.001-10 g/kg.

The pharmaceutical composition of the present disclosure may be formulated into a single-dose or multiple-dose form using a pharmaceutically acceptable carrier and/or excipient. The formulation may be a solution in an oily or aqueous medium, a suspension, an emulsion, an extract, a powder, a granule, a tablet or a capsule, and may further contain a dispersant or a stabilizer.

The present disclosure also provides a functional health food composition for preventing, ameliorating or treating sleep disorder, which contains the extract of the roasted citrus byproduct as an active ingredient. The functional health food refers to a food prepared into a capsule, a powder, a suspension, etc. by adding the extract of the roasted citrus byproduct to various food materials, which provides specific health benefit without side effects that may occur when a pharmaceutical drug is taken for a long time because food is used as a raw material. The functional health food of the present disclosure is very useful since it can be taken routinely. Although the addition amount of the extract of the roasted citrus byproduct in the functional health food can vary depending on the type of the functional health food, it is not specially limited as long as the taste of the food is not sacrificed. The addition amount is generally 0.01-50 wt%, specifically 0.1-20 wt%. For a functional health food in the form of a pill, a granule, a tablet or a capsule, the addition amount is generally 0.1-100 wt% specifically 0.5-80 wt%. In a specific exemplary embodiment, the functional health food of the present disclosure may be in the form of a powder, a granule, a tablet, a capsule or a beverage.

The present disclosure also provides a use of the extract of the roasted citrus byproduct for preparation of a drug or a food for preventing, ameliorating or treating sleep disorder. As described above, the extract of the roasted citrus byproduct may be used for preventing, ameliorating or treating sleep disorder or insomnia.

The present disclosure also provides a method for preventing, ameliorating or treating sleep disorder or insomnia, which includes administering an effective amount of the extract of the roasted citrus byproduct to a mammal.

As used herein, the term "mammal" refers to a mammal which is a subject of treatment, monitoring or experiment, specifically human.

As used herein, the term "effective amount" refers to an amount of the active ingredient or the pharmaceutical composition that will elicit a biological or medical response in a tissue system, an animal or human that is being sought by a researcher, a veterinarian, a medical doctor or a clinician, including alleviation of the symptoms of the corresponding disease or disorder. The effective amount of the active ingredient of the present disclosure and the number of administration thereof will vary depending on the desired effect. Accordingly, the optimum administration dose may be easily determined by those skilled in the art and it may be adjusted depending on various factors including the particular disease, the severity of the disease, the contents of the active ingredient and other ingredients contained in the composition, formulation type, the age, body weight, general physical conditions, sex and diet of a patient, administration time, administration route, the excretion rate of the composition, treatment period and drug(s) used in combination. In the method for preventing, ameliorating or treating of the present disclosure, a preferred daily dose of the extract of the roasted citrus byproduct for an adult is 0.001-10 g/kg, once or several times a day.

The composition containing the extract of the roasted citrus byproduct as an active ingredient may be administered orally, rectally, intravenously, intraarterially, intraperitoneally, intramuscularly, intrasternally, transdermally, topically, intraocularly or intradermally.

The present disclosure also provides a method for preparing a citrus byproduct extract with enhanced effect of preventing or alleviating sleep disorder or insomnia, which includes: (1) a step of drying and pulverizing a byproduct of a citrus; (2) a step of roasting the pulverization product of the citrus byproduct; (3) a step of extracting the roasted pulverization product of the citrus byproduct with a C₁₋₆ lower alcohol or an aqueous solution thereof; and (4) a step of filtering and concentrating the extract of the citrus byproduct.

The matters described above with regard to the "citrus byproduct extract" and the "effect of preventing or alleviating sleep disorder or insomnia" will be omitted to avoid unnecessary redundancy.

Specifically, the byproduct of a citrus may be a byproduct obtained after extracting oil ingredients from the rind of the citrus in terms of sleep efficiency.

Hereinafter, the composition for preventing, ameliorating or treating sleep disorder or insomnia, which contains the extract of the roasted citrus byproduct as an active ingredient, according to the present disclosure will be described in detail referring to examples and comparative examples.

### <Examples>

### Example 1: Roasted lime rind ethanol extract (Lime)

(1) First, a byproduct obtained by extracting oil ingredients from lime rind was prepared (Citro Jugo S.A., Mexico). A lime rind powder (water content: 4.5 wt%) was prepared by drying and pulverizing the byproduct.
(2) The lime rind powder was put in a Barwell coffee roaster (SCR-301, China) and roasted at 200 °C for 15 minutes.
(3) After mixing the roasted lime rind powder with a 70% (v/v) ethanol aqueous solution at a weight ratio of 1 : 10, a roasted lime rind ethanol extract was obtained by extracting while stirring at 50 °C for 16 hours at 150 rpm.
(4) The obtained extract was filtered, concentrated under reduced pressure, and then freeze-dried into a powder.

### Example 2: Roasted lemon rind ethanol extract (Lemon)

A roasted lemon rind ethanol extract was prepared in the same manner as in Example 1 using a lemon rind byproduct (Citromsx, Argentina) instead of the lime rind byproduct.

### Example 3: Roasted kumquat rind ethanol extract (Man)

A roasted kumquat rind ethanol extract was prepared in the same manner as in Example 1 using a kumquat rind byproduct instead of the lime rind byproduct.

### Example 4: Ethanol extract of roasted lime rind (30%)

A roasted lemon rind ethanol extract was prepared in the same manner as in Example 1 using a 30% ethanol aqueous solution instead of the 70% ethanol aqueous solution.

### Example 5: Ethanol extract of roasted lemon rind (30%)

A roasted lemon rind ethanol extract was prepared in the same manner as in Example 2 using a 30% ethanol aqueous solution instead of the 70% ethanol aqueous solution.

### Comparative Example 1-1: Ethanol extract of unroasted lime rind

A lime rind ethanol extract was prepared in the same manner as in Example 1 without roasting.

### Comparative Example 1-2: Ethanol extract of unroasted lemon rind

A lemon rind ethanol extract was prepared in the same manner as in Example 2 without roasting.

### Comparative Example 1-3: Ethanol extract of unroasted kumquat rind

A kumquat rind ethanol extract was prepared in the same manner as in Example 3 without roasting.

### Comparative Example 2-1: Hot water extract of roasted lime rind

A roasted lime rind hot water extract was prepared in the same manner as in Example 1 by conducting hot water extraction at 100 °C using distilled water instead of ethanol extraction at 50 °C using a 70% ethanol aqueous solution.

### Comparative Example 2-2: Hot water extract of roasted lemon rind

A roasted lemon rind hot water extract was prepared in the same manner as in Example 2 by conducting hot water extraction at 100 °C using distilled water instead of ethanol extraction at 50 °C using a 70% ethanol aqueous solution.

### Comparative Example 3-1: Hot water extract of unroasted lime rind

A lime rind hot water extract was prepared in the same manner as in Comparative Example 2-1 without roasting.

### Comparative Example 3-2: Hot water extract of unroasted lemon rind

A lemon rind hot water extract was prepared in the same manner as in Comparative Example 2-2 without roasting.

### <Test methods>

### Experimental animals

ICR mice (18-22 g, male) and C57BL/6N mice (28-30 g, male) acquired from Koatech (Pyeongtaek, Gyeonggi-do, Korea) were accommodated to the experimental environment for a week. They were housed in cages maintained at a temperature of 25 ± 1 °C and a humidity of 65 ± 5%, with a light/dark cycle (lighting from 7 a.m. to 7 p.m., 3000 lux). They had free access to feed and water. All the animals were cared for in accordance with the guidelines of Pukyong University Animal Care Committee.

### Pentobarbital-induced sleep test

Pentobarbital-induced sleep test was carried out at regular regulars between 1 p.m. and 5 p.m. Ten (n = 10) mice per each group were fasted for 24 hours before the test. All test samples were prepared using a 5% Tween 80-saline solution and administered orally (p.o.) to the mice 45 minutes before the administration of pentobarbital. A normal control group was treated with a 5% Tween 80-saline aqueous solution at a concentration of 10 mg/kg. Doxepin hydrochloride (Hanmi Pharm) and diazepam (Myung In Pharm), which are representative sleeping drugs, were used as positive control group drugs for comparison of sleep-improving effect with the extract. Pentobarbital (Hanlim Pharm) was administered intraperitoneally (i.p.) at a concentration of 45 mg/kg (hypnotic dosage) according to the experimental design. After the pentobarbital treatment, the mice were transferred to separated spaces and sleep latency and sleep duration were measured. The sleep latency was determined by the time until the righting reflex was lost for 1 minute or longer after the intraperitoneal injection of pentobarbital, and the sleep duration was determined by the time until the righting reflex was restored. The mice which showed no sleeping behavior even 10 minutes after the administration of pentobarbital were excluded from the test.

### Sleep architecture analysis

After the accommodation period of 1 week, electrodes were inserted into the C57BL/6N mice for measurement of electroencephalograms (EEGs) and electromyograms (EMGs). After anesthetizing the mouse with pentobarbital (50 mg/kg, i.p.), the head was fixed in a stereotaxic instrument. After incising the subcutaneous connective tissue, a mouse EEG/EMG Headmount (Pinnacle Technology Inc, Oregon, USA) was inserted for EEG and EMG recording. After fixing with dental cement followed by suturing and disinfection, antibiotics were injected for 3 days to prevent inflammation. The mice were allowed to recover for 7 days. For accommodation to the measurement environment, the recording apparatus was connected from 4 days prior to the measurement. After orally administering the sample and waiting for 5 minutes for sedation, EEG and EMG were recorded for 24 hours from 17:00 using PAL-8200 (Pinnacle Technology Inc., Oregon, USA). Sampling rate was set at 200 Hz (epoch time: 10 s). Filtering range was 0.1-25 Hz for EEG and 10-100 Hz for EMG. Sleep architecture was analyzed according to the fast Fourier transform (FFT) algorithm using SleepSign (Ver. 3.0, Kissei Comtec, Nagono, Japan). The result was represented by dividing into wake (wakefulness), REMS (rapid eye movement sleep (theta band: 6-10 Hz) and NREMS (non-rapid eye movement sleep (delta band: 0.65-4 Hz). Sleep latency was determined by the time until non-REM sleep with 10-sec epoch occurred consecutively at least 12 times.

### <Test Examples>

### Test Example 1: Analysis of ingredients and size of raw materials

The contents and size distribution of the ingredients of the lime rind powder prepared in the step (1) of Example 1 was analyzed. The result is shown below in Table 1 and Table 2.

In Table 1, the water content was measured by the method described in Official Methods of Analysis of the AOAC (1990), 15th Ed., Association of Official Analytical Chemists, Arlington, VA. USA.

The content of oil ingredients was measured according to the method described in Dann A. Kimball (1999). Citrus Processing; a complete guide. United States of America: Denisse Hawkins Coursey (221-239) and Analysis method IFU No. 45 (rev. 2005). Determination of essential.

And, the content of water-soluble ingredients was measured according to the method described in AOAC, 14th Edition. 1984. 22.024 Determination of Solids Soluble in Fruits and Fruit Products Refractometer Method.

**[Table 1]**

| | Contents |
|---|---|
| Water content | 10.01 wt% |
| Content of oil ingredients | 0.08 wt% |
| Content of water-soluble ingredients | 9.48 wt% |

From Table 1, it can be seen that the lime rind powder prepared in the step (1) of Example 1 contains only 0.08 wt% of oil ingredients because it is the powder of a byproduct remaining after extracting oil ingredients from the lime rind.

**[Table 2]**

| | | Contents |
|---|---|---|
| Particle size distribution (100 wt% in total) | 4-15 mm | 18.00 wt% |
| | 1-4 mm | 76.00 wt% |
| | < 1 mm | 6.00 wt% |

From Table 2, it can be seen that the lime rind powder prepared in the step (1) of Example 1 is a powder with a size of 15 mm or smaller, mostly in a range from 1 mm to 4 mm.

### Test Example 2: Pentobarbital-induced sleep test using citrus byproduct extracts

### 2-1: Sleep efficiency of extracts of Examples 1 and 2

Pentobarbital-induced sleep test was carried out to investigate the effect of the citrus byproduct extracts of Example 1 and Example 2 on sleep efficiency. For this, mice were divided into 10 groups of 10 mice per each. 5% Tween 80-saline was administered to a normal control group and 30 mg/kg doxepin hydrochloride (DH) was administered to a positive control group. And, the roasted lime rind ethanol extract of Example 1 (Lime) and the roasted lemon rind ethanol extract of Example 2 (Lemon) were administered orally (p.o.) at 100 mg/kg and 300 mg/kg, respectively. Then, the sleep-inducing effect by pentobarbital (45 mg/kg, i.p.) was investigated.

As a result, the roasted lime rind ethanol extract of Example 1 (Lime) and the roasted lemon rind ethanol extract of Example 2 (Lemon) showed significant sleep latency-decreasing effect (see FIG. 2a) and sleep duration-increasing effect (see FIG. 2b) at relatively low concentrations of 100 mg/kg and 300 mg/kg for plant extracts. In particular, the roasted lime rind ethanol extract of Example 1 showed further improved sleep efficiency as compared to doxepin hydrochloride, which is a commercially available sleeping drug.

### 2-2: Comparison of sleep efficiency of extracts of examples and comparative examples

Pentobarbital-induced sleep test was carried out to investigate the effect of the citrus byproduct extracts of Example 1 and Example 2 on sleep efficiency. For this, mice were divided into 10 groups of 10 mice per each. 5% Tween 80-saline was administered to a normal control group and 30 mg/kg doxepin hydrochloride (DH) was administered to a positive control group. And, the roasted lime rind ethanol extract of Example 1 (Lime, 100 mg/kg), the lime rind extracts of Comparative Example 1-1, Comparative Example 2-1 and Comparative Example 3-1 (100 mg/kg), the roasted lemon rind ethanol extract of Example 2 (Lemon, 300 mg/kg) and the lemon rind extracts of Comparative Example 1-2, Comparative Example 2-2 and Comparative Example 3-2 (300 mg/kg) were administered orally (p.o.). Then, the sleep-inducing effect by pentobarbital (45 mg/kg, i.p.) was investigated.

As a result, the roasted lime rind ethanol extract of Example 1 (Lime, EtOH + roasting) and the unroasted lime rind ethanol extract of Comparative Example 1-1 (EtOH) showed significantly decreased sleep latency and significantly increased sleep duration as compared to the control group (CON). In particular, the roasted lime rind ethanol extract of Example 1 showed significantly decreased sleep latency and significantly increased sleep duration as compared to the unroasted lime rind ethanol extract of Comparative Example 1-1 (see FIG. 3a).

In addition, the roasted lemon rind ethanol extract of Example 2 (Lemon, EtOH + roasting) and the unroasted lemon rind ethanol extract of Comparative Example 1-2 (EtOH) showed significantly decreased sleep latency and significantly increased sleep duration as compared to the control group (CON). In particular, the roasted lemon rind ethanol extract of Example 2 showed significantly decreased sleep latency and significantly increased sleep duration as compared to the unroasted lemon rind ethanol extract of Comparative Example 1-2 (see FIG. 3b).

### 2-3: Sleep efficiency of extracts of examples

Pentobarbital-induced sleep test was carried out to investigate the effect of the citrus byproduct extracts of Examples 1, 2, 4 and 5 on sleep efficiency. For this, mice were divided into 10 groups of 10 mice per each. 5% Tween 80-saline was administered to a normal control group and 30 mg/kg doxepin hydrochloride (DH) was administered to a positive control group. And, the roasted lime rind ethanol extracts of Example 1 and Example 4 (Lime, 100 mg/kg) and the roasted lemon rind ethanol extracts of Example 2 and Example 5 (Lemon, 300 mg/kg) were administered orally (p.o.). Then, the sleep-inducing effect by pentobarbital (45 mg/kg, i.p.) was investigated. The result is shown in Table 3.

**[Table 3]**

| | | Sleep latency (min) | Sleep duration (min) |
|---|---|---|---|
| Control group | | 3.5 | 72.1 |
| Doxepin hydrochloride (DH) | | 2.9*** | 121.2*** |
| Lime | Example 1 | 2.7*** | 161.7*** |
| Lime | Example 4 | 3.1** | 148.5*** |
| Lemon | Example 2 | 3.0*** | 125.4*** |
| Lemon | Example 5 | 3.3* | 117.6*** |

As seen from Table 3, the roasted citrus byproduct extracts of Examples 1, 2, 4 and 5 showed significantly increased sleep duration at relatively low concentrations of 100 mg/kg and 300 mg/kg. In particular, the roasted lime rind ethanol extracts of Example 1 and Example 4 showed remarkably improved sleep efficiency as compared to doxepin hydrochloride, which is a commercially available sleeping drug.

### Test Example 3: Comparison of sleep efficiency depending on roasting

Pentobarbital-induced sleep test was carried out to investigate the effect of the citrus byproduct extracts of Example 3 and Comparative Examples 1-3 on sleep efficiency. For this, mice were divided into 2 groups of 10 mice per each. 5% Tween 80-saline was administered to a normal control group. And, the roasted kumquat rind ethanol extract of Example 3 (Roasting O, 200 mg/kg) and the kumquat rind ethanol extracts of Comparative Examples 1-3 (Roasting X, 200 mg/kg) were administered orally (p.o.). Then, the sleep-inducing effect by pentobarbital (45 mg/kg, i.p.) was investigated.

As a result, the roasted kumquat rind ethanol extract of Example 3 (Roasting O) showed significant sleep latency-decreasing effect (see FIG. 4a) and sleep duration-increasing effect (see FIG. 4b) at a low concentration of 200 mg/kg for a plant extract. In contrast, the kumquat rind ethanol extracts of Comparative Examples 1-3 (Roasting X) did not show significant sleep latency-decreasing effect and the sleep duration-increasing effect was also remarkably lower as compared to the extract of Example 2.

From this result, it can be seen that roasting is an essential process for improving sleep efficiency in the method for preparing a citrus byproduct extract according to the present disclosure.

FIG. 5 briefly compares the amount of the raw material used in the method of preparing an extract according to an exemplary embodiment of the present disclosure with the amount of the raw material used in the method of Comparative Example 1 without roasting.

Referring to FIG. 5, it can be seen that the same amount of raw material is used in both the method of preparing an extract according to an exemplary embodiment of the present disclosure and the method of Comparative Example 1 without roasting. Accordingly, the difference in sleep efficiency is not due to the difference in the amount of the raw material used.

### Test Example 4: Analysis of sleep architecture using roasted citrus byproduct extracts

Sleep architecture analysis was conducted to evaluate the effect of the roasted citrus byproduct extracts according to an exemplary embodiment of the present disclosure on the quantity and quality of sleep. For this, mice were divided into 4 groups of 8 mice per each. 5% Tween 80-saline was administered to a normal control group and 10 mg/kg zolpidem (ZPD) was administered to a positive control group. And, the sleep architecture of mice was analyzed after orally administering (p.o.) the roasted lime rind ethanol extract of Example 1 (Lime, 100 mg/kg) and the roasted lemon rind ethanol extract of Example 2 (Lemon, 300 mg/kg).

As a result, the extracts of Example 1 and Example 2 exhibited sleep efficiency comparable to that of zolpidem. Specifically, sleep latency was decreased significantly, non-REM sleep was increased and wake was decreased, as compared to the control group (see FIGS. 6a and 6b).

In addition, after oral administration the roasted lime rind ethanol extract of Example 1 (Lime, 100 mg/kg), the roasted lemon rind ethanol extract of Example 2 (Lemon, 300 mg/kg) or zolpidem (ZPD, 10 mg/kg), the change in wake, NREM sleep and REM sleep was measured while measuring the brain waves of C57BL/6N mice. As shown in FIG. 6c, the extract of Example 1 showed significantly increased NREM sleep for the first 2 hours after the oral administration, and the extract of Example 2 showed significantly increased NREM sleep for the first 1 hour after the oral administration. The positive control group, i.e., zolpidem, showed significantly increased NREM sleep for the first 6 hours after the oral administration. From this result, it can be seen that, whereas the extracts of Example 1 and Example 2 induced spontaneous sleeping after exhibiting sleep-enhancing effect in the early stage of sleep, the sleep-enhancing effect of the sleeping drug zolpidem was continued for a long time, which is a side effect caused by the sleeping drug.

The change in delta activity during non-REM sleep can be seen as a physiological indicator of sleep quality and depth [Behav. Brain Res. 210, 5456. Huang et al., 2010]. It is also reported that zolpidem increases non-REM sleep and decreases delta power during non-REM sleep [PNAS. 101, 3674-3679. Kopp et al., 2004]. These typical characteristics of zolpidem were verified clearly through experiment. Zolpidem significantly decreased delta activity during non-REM sleep (p < 0.01) as expected, whereas the extracts of Example 1 and Example 2 according to the present disclosure did not show significant change in delta activity (see FIG. 7). This result suggests that the extracts according to the examples of the present disclosure increase NREM sleep without affecting physiological sleep.

An ideal sleeping drug should exhibit fast sleep latency and continued sleep duration without affecting physiological sleep [Sleep. 31, 259-270. Alexandre et al., 2008]. Accordingly, it is thought that the extract of the roasted citrus byproduct of the present disclosure can enhance sleep more safely as compared to the BDZ and non-BDZ drugs having side effects such as zolpidem.

### Test Example 5: Sleep-improving mechanism of roasted citrus byproduct extract

The sleep-improving mechanism of the roasted citrus byproduct extract was investigated by transforming cells with recombinant GABA subunits and primarily cultured neurons.

First, HEK293T cells (human embryonic kidney 293 cells) were cultured by adding 10% FBS (fetal bovine serum) and 1% penicillin-streptomycin to DMEM (Dulbecco's modified Eagle's medium). The cultured HEK293T cells were treated with the cDNAs of α1, β3 and γ2 subunits at 1:1:1 using a transfection reagent (QIAGEN) for transformation with the recombinant GABA receptors. The expression of the GABA receptors in the HEK293T cells was investigated using an AAV-GFP plasmid vector in which the α1 cDNA was sub-cloned.

An Axon 700B patch-clamp amplifier (Molecular device, Foster City, CA, USA), a Digidata 1440 interface, and a Clampex and Clampfit software (Version10, Molecular Device) were used.

The activity of the GABA receptors in the HEK-293T cells transformed with the recombinant GABA receptors was investigated depending on the concentration of the roasted lime rind ethanol extract of Example 1 (N1 sample).

In order to measure the activity of the GABA receptors, current flow through the receptors was measured in the HEK-293T cells with the expression of the GABA receptor confirmed by optical microscopy in a voltage clamp mode. As a control group, after treating the cells with 1 µM GABA for 3 seconds, the magnitude of induced GABA-mediated current was recorded. After measuring the magnitude of induced current while treating with the N1 sample at 0.1, 1, 10 or 100 µg/mL together with GABA, the activity was compared with the control group (FIG. 8). Then, statistical significance for the results was tested by t-test.

Referring to FIG. 8, it can be seen that the roasted lime rind ethanol extract of Example 1 increases the activity of the GABA receptors in a concentration-dependent manner.

In addition, neurons were cultured primarily in order to investigate whether the increased activity of the GABA receptors by the roasted lime rind ethanol extract of Example 1 (N1 sample) is associated with the GABA-binding site or the benzodiazepam site.

Specifically, neurons were isolated from the brain of a P0 mouse and cultured in a neurobasal medium supplemented with 2% B-27, 2 mM L-glutamine and 20 IU/mL pen-strep. After culturing the cells for 3 weeks while replacing with a fresh medium every 3 days, the activity of the GABA receptor was investigated by an electrophysiolgical method.

An Axon 700B patch-clamp amplifier (Molecular device, Foster City, CA, USA), a Digidata 1440 interface, and a Clampex and Clampfit software (Version10, Molecular Device) were used.

In order to investigate whether the increased activity of the GABA receptors by the roasted lime rind ethanol extract of Example 1 (N1 sample) is associated with the GABA-binding site or the benzodiazepam site, current flow through the receptors was measured in the cultured neurons in a voltage clamp mode. As a control group, after treating the cells with 1 µM GABA for 3 seconds, the magnitude of induced GABA-mediated current was recorded. After measuring the magnitude of induced current while treating with the N1 sample at 10 µg/mL (N1 10), the N1 sample at 10 µg/mL and 3 µM flumazenil (N1 10 + FLZ), or the N1 sample at 10 µg/mL, 3 µM flumazenil and 10 µM bicuculline (BIC) (N1 10 + FLZ + BIC), the activity was compared with the control group (FIG. 9). Then, statistical significance for the results was tested by t-test.

Referring to FIG. 9, it can be seen that the roasted lime rind ethanol extract of Example 1 significantly increased the activity of the GABA receptors. The increase was slightly inhibited by flumazenil, which is an antagonist against the benzodiazepine receptor, but the increase was not significant. Meanwhile, the increased activity of the GABA receptors by the roasted lime rind ethanol extract of Example 1 was decreased remarkably by the addition of bicuculline, which is an antagonist of GABA receptors. Therefore, it can be seen that the roasted lime rind ethanol extract of Example 1 acts directly on the GABA receptors.

### Test Example 6: Analysis of contents of main ingredients

### 6-1: Analysis of limonene content

The content of limonene in the extracts of the examples and comparative examples was investigated by SPME (solid-phase microextraction).

Specifically, 0.5 mL of a solution obtained by dissolving 20 mg of the extract of the examples or comparative examples in 100 mL of distilled water was analyzed using an Agilent 7890A GC instrument (mode: splitless) equipped with an Agilent DB-FFAP column (60 m × 0.26 mm × 0.5 µm). An injection volume of 0.5 mL was flown at a rate of 17 mL/min. The conditions of SPME were as follows: adsorption time = 15 minutes, adsorption temperature = 25 °C, oven heating at 5 °C/min from 60 °C (hold for 3 minutes) to 220 °C (hold for 15 minutes). Sample injection temperature and detection temperature were maintained at 240 °C. The data of the sample solution obtained by SPME were compared with the calibration curve for the standard. The result of calculating the limonene content for the examples and comparative examples is given in Table 4.

**[Table 4]**

| | Limonene (g/100g) |
|---|---|
| Example 1 | 18.7 |
| Example 2 | 22.4 |
| Example 3 | 17.2 |
| Comparative Example 1-1 | 17.6 |
| Comparative Example 1-2 | 20.3 |
| Comparative Example 1-3 | 16.5 |

As seen from Table 4, there was no significant difference in the limonene content between the extracts of the roasted citrus byproducts of the examples and the extracts of the unroasted citrus byproducts of the comparative examples.

### 6-2: Analysis of contents of narirutin and hesperidin

The contents of flavonoid compounds contained in the extracts of the examples and comparative examples were investigated.

Specifically, a solution obtained by dissolving 20 mg of the extract of the examples or comparative examples in 100 mL of distilled water was analyzed using a reverse-phased HPLC system (Vltimate 3000; Thermo Fisher Scientific, Waltham, USA) consisting of an autosampler, a binary pump and a PDA detector. A C18 reverse-phased HPLC column (Discovery C18, 5 µm, 4.6 × 250 mm; Sigma, MO, USA) was used. An injection volume of 10 µL was flown at a rate of 0.8 mL/min.

100% triply distilled water and 100% acetonitrile were used as solvents (mobile phases) A and B in the HPLC, respectively. The gradient elution was 90% A/10% B for 0-5 minutes, 75% A/25% B for 5-14 minutes, 40% A/60% B for 14-25.5 minutes, 20% A/80% B for 25.5-30.5 minutes, and 90% A/10% B for 30.5-35 minutes. All the compounds were detected at a wavelength of 280 nm and the analysis temperature was maintained at 40 °C. The data of the sample solution obtained by HPLC/PDA were compared with the retention time and UV spectrum for the standard. The result of calculating the narirutin and hesperidin contents for the examples and comparative examples is given in Table 5.

**[Table 5]**

| | Narirutin (g/100 g) | Hesperidin (g/100 g) |
|---|---|---|
| Example 1 | 34.7 | 10.6 |
| Example 2 | 29.4 | 9.7 |
| Example 3 | 28.6 | 9.1 |
| Comparative Example 1-1 | 31.8 | 10.8 |
| Comparative Example 1-2 | 27.6 | 9.2 |
| Comparative Example 1-3 | 27.9 | 8.8 |

Referring to Table 5, there was no significant difference in the narirutin and hesperidin contents between the extracts of the roasted citrus byproducts of the examples and the extracts of the unroasted citrus byproducts of the comparative examples.

These results mean that the sleep efficiency of the extracts of the roasted citrus byproducts of the examples is not due to limonene, narirutin or hesperidin.

### 6-3: Analysis of vicenin content

The content of vicenin in the extracts of the examples and comparative examples was investigated by liquid chromatography (RI).

Specifically, a solution obtained by dissolving 10 mg of the extract of the examples or comparative examples in 10 mL of a solvent was analyzed using a Hitach Chromaster CM5000 Series refractive index detector equipped with a symmetry C18 column (4.6 mm × 250 mm WAT054275). The solvent was a simple mixture of 50% DMSO and 50% methanol. 10 µL of the sample was injected at a rate of 0.7 mL/min.

1% acetic acid and 100% acetonitrile were used as mobile phases A and B in the liquid chromatography, respectively. The gradient elution was 90% A/10% B for 0-10 minutes, 90% A/10% B for 10-15 minutes, 70% A/30% B for 15-17.9 minutes, 70% A/30% B for 17.9-18 minutes, 55% A/45% B for 18-25 minutes, 55% A/45% B for 25-30 minutes, 30% A/70% B for 30-35 minutes, 0% A/100% B for 35-40 minutes, and 90% A/10% B for 40-45 minutes. Vicenin was detected at a wavelength of 320 nm and the analysis temperature was maintained at 40 °C.

The obtained data of the sample solution were compared with the retention time and UV spectrum for the standard. The result of calculating the vicenin content for the examples and comparative examples is given in Table 6.

**[Table 6]**

| | Vicenin (mg/L) |
|---|---|
| Example 1 | 6.2 |
| Example 2 | 6.0 |
| Example 3 | 5.4 |
| Comparative Example 1-1 | 1.2 |
| Comparative Example 1-2 | 0.8 |
| Comparative Example 1-3 | 1.0 |

Referring to Table 6, the vicenin content was significantly higher in the extracts of the roasted citrus byproducts of the examples as compared to the extracts of the unroasted citrus byproducts of the comparative examples.

### 6-4: Analysis of sleep efficiency of vicenin

Pentobarbital-induced sleep test was performed to investigate the effect of vicenin on sleep efficiency. For this, mice were prepared with 10 mice per group. 5% Tween 80-saline was administered to a normal control group and 30 mg/kg doxepin hydrochloride (DH) was administered to a positive control group. The sleep-inducing effect by pentobarbital (45 mg/kg, i.p.) was analyzed after orally administering (p.o.) vicenin at 6.25, 12.5, 25 or 50 mg/kg.

As a result, vicenin showed significant sleep latency-decreasing effect (see FIG. 10a) and sleep duration-increasing effect (see FIG. 10b) at a concentration of 50 mg/kg.

Hereinafter, formulation examples of a composition containing an extract of a roasted citrus byproduct of the present disclosure are described. However, they are provided to describe the present disclosure specifically not to limit the present disclosure.

### Formulation Example 1. Preparation of powder

| | |
|---|---|
| Extract powder obtained in Example 1 or 2 | 300 mg |
| Lactose | 100 mg |
| Talc | 10 mg |

A powder was prepared by mixing the above ingredients and filling in an airtight pouch.

### Formulation Example 2. Preparation of tablet

| | |
|---|---|
| Extract powder obtained in Example 1 or 2 | 300 mg |
| Cornstarch | 100 mg |
| Lactose | 100 mg |
| Magnesium stearate | 2 mg |

After mixing the above ingredients, a tablet was prepared by a common method.

### Formulation Example 3. Preparation of capsule

| | |
|---|---|
| Extract powder obtained in Example 1 or 2 | 300 mg |
| Crystalline cellulose | 3 mg |
| Lactose | 14.8 mg |
| Magnesium stearate | 0.2 mg |

After mixing the above ingredients, a capsule was prepared by filling in a gelatin capsule by a common method.

### Formulation Example 4. Preparation of injection

| | |
|---|---|
| Extract powder obtained in Example 1 or 2 | 600 mg |
| Mannitol | 180 mg |
| Sterilized distilled water for injection | 2974 mg |
| Na₂HPO₄·12H₂O | 26 mg |

An injection was prepared by a common method with the contents described above per ampoule.

### Formulation Example 5. Preparation of liquid formulation

| | |
|---|---|
| Extract powder obtained in Example 1 or 2 | 4 g |
| High-fructose corn syrup | 10 g |
| Mannitol | 5 g |
| Purified water | Adequate |

After dissolving the above ingredients in purified water and adding an adequate amount of lemon flavor, purified water was added such that the total amount was 100 g. The prepared liquid formulation was filled in a brown bottle and then sterilized.

### Formulation Example 6. Preparation of granule

| | |
|---|---|
| Extract powder obtained in Example 1 or 2 | 1,000 mg |
| Vitamin mixture | Adequate |
| Vitamin A acetate | 70 µg |
| Vitamin E | 1.0 mg |
| Vitamin B₁ | 0.13 mg |
| Vitamin B₂ | 0.15 mg |
| Vitamin B₆ | 0.5 mg |
| Vitamin B₁₂ | 0.2 µg |
| Vitamin C | 10 mg |
| Biotin | 10 µg |
| Nicotinamide | 1.7 mg |
| Folic acid | 50 µg |
| Calcium pantothenate | 0.5 mg |
| Mineral mixture | Adequate |
| Ferrous sulfate | 1.75 mg |
| Zinc oxide | 0.82 mg |
| Magnesium carbonate | 25.3 mg |
| Monopotassium phosphate | 15 mg |
| Dicalcium phosphate | 55 mg |
| Potassium citrate | 90 mg |
| Calcium carbonate | 100 mg |
| Magnesium chloride | 24.8 mg |

The compositions of the vitamin and mineral mixtures described above are provided as relatively appropriate examples for a granule but may be varied as desired. After preparing a granule by mixing the above ingredients according to a common method, it was used to prepare a functional health food composition by a common method.

### Formulation Example 7. Preparation of functional beverage

| | |
|---|---|
| Extract powder obtained in Example 1 or 2 | 1,000 mg |
| Citric acid | 1,000 mg |
| Oligosaccharide | 100 g |
| Plum concentrate | 2 g |
| Taurine | 1 g |
| Purified water | To 900 mL |

After mixing the above ingredients and stirring for about 1 hour while stirring at 85 °C, the resulting solution was filtered and collected in a sterilized 2-L container according to a common method, which was then sealed, sterilized and kept in a refrigerator for use in preparing a functional beverage composition of the present disclosure.

The above-described composition is provided as a relatively appropriate example for a preference beverage but may be varied as desired in consideration of the regional and ethnic preferences of consumers and countries, purpose of use, etc.

Although the specific exemplary embodiments of the present disclosure have described above, various changes or modifications can be made thereto without departing from the scope of present disclosure. In addition, the appended claims encompass such changes or modifications encompassed within the scope of the present disclosure.

## Claims

1. A food composition for preventing or alleviating sleep disorder or insomnia, comprising an extract obtained by extracting a roasted citrus byproduct with a C₁₋₆ lower alcohol or an aqueous solution thereof as an active ingredient.

2. The food composition for preventing or alleviating sleep disorder or insomnia according to claim 1, wherein the citrus is one or more selected from lemon, lime, tangerine, hallabong, cheonhyehyang, green tangerine, kumquat, orange, grapefruit, hardy orange and citron.

3. The food composition for preventing or alleviating sleep disorder or insomnia according to claim 1, wherein the roasting is performed at 150-230 °C for 5-20 minutes.

4. The food composition for preventing or alleviating sleep disorder or insomnia according to claim 1, wherein the citrus byproduct is one or more selected from the rind and pomace of a citrus.

5. The food composition for preventing or alleviating sleep disorder or insomnia according to claim 1, wherein the extract of the citrus byproduct is obtained by extracting with 10-90 vol% methanol, ethanol, butanol, propanol or an aqueous solution thereof.

6. The food composition for preventing or alleviating sleep disorder or insomnia according to claim 1, wherein the extract of the citrus byproduct is obtained by extracting with a 50-80 vol% ethanol aqueous solution.

7. The food composition for preventing or alleviating sleep disorder or insomnia according to claim 1, wherein the extract of the citrus byproduct is obtained by extracting at 10-80 °C.

8. The food composition for preventing or alleviating sleep disorder or insomnia according to claim 1, wherein the extract of the citrus byproduct is prepared by a method comprising:
(1) a step of drying and pulverizing a byproduct of a citrus;
(2) a step of roasting the pulverization product of the citrus byproduct;
(3) a step of extracting the roasted pulverization product of the citrus byproduct with a C₁₋₆ lower alcohol or an aqueous solution thereof; and
(4) a step of filtering and concentrating the extract of the citrus byproduct.

9. The food composition for preventing or alleviating sleep disorder or insomnia according to claim 8, wherein the byproduct of a citrus in the step (1) is a byproduct obtained after extracting oil ingredients from the rind of a citrus.

10. The food composition for preventing or alleviating sleep disorder or insomnia according to claim 1, wherein the prevention or alleviation of sleep disorder or insomnia is for decreasing sleep latency, increasing sleep duration or increasing non-REM sleep.

11. The food composition for preventing or alleviating sleep disorder or insomnia according to claim 1, wherein the food composition is in the form of a powder, a granule, a tablet, a capsule or a beverage.

12. A pharmaceutical composition for preventing or treating sleep disorder or insomnia, comprising an extract obtained by extracting a roasted citrus byproduct with a C₁₋₆ lower alcohol or an aqueous solution thereof as an active ingredient.

13. The pharmaceutical composition for preventing or treating sleep disorder or insomnia according to claim 12, wherein the roasting is performed at 150-230 °C for 5-20 minutes.

14. The pharmaceutical composition for preventing or treating sleep disorder or insomnia according to claim 12, wherein the extract of the citrus byproduct is prepared by a method comprising:
(1) a step of drying and pulverizing a byproduct of a citrus;
(2) a step of roasting the pulverization product of the citrus byproduct;
(3) a step of extracting the roasted pulverization product of the citrus byproduct with a C₁₋₆ lower alcohol or an aqueous solution thereof; and
(4) a step of filtering and concentrating the extract of the citrus byproduct.

15. The pharmaceutical composition for preventing or treating sleep disorder or insomnia according to claim 14, wherein the byproduct of a citrus in the step (1) is a byproduct obtained after extracting oil ingredients from the rind of a citrus.

16. The pharmaceutical composition for preventing or treating sleep disorder or insomnia according to claim 12, wherein the prevention or treatment of sleep disorder or insomnia is for decreasing sleep latency, increasing sleep duration or increasing non-REM sleep.

17. A method for preparing a citrus byproduct extract with enhanced effect of preventing or alleviating sleep disorder or insomnia, comprising:
(1) a step of drying and pulverizing a byproduct of a citrus;
(2) a step of roasting the pulverization product of the citrus byproduct;
(3) a step of extracting the roasted pulverization product of the citrus byproduct with a C₁₋₆ lower alcohol or an aqueous solution thereof; and
(4) a step of filtering and concentrating the extract of the citrus byproduct.
